# EUROPEAN PATENT SPECIFICATION

(11) **EP 0 295 817 B1**
(45) Date of publication and mention of the grant of the patent: **05.05.1993**
(21) Application number: 88305119.5
(22) Date of filing: 03.06.1988
(51) Int. Cl.: A61F 5/44

(54) **Securing an outlet pipe or tap to a liquid-containing bag**
Befestigen eines Auslassrohrs oder -hahns an einen eine Flüssigkeit enthaltenden Beutel
Fixation d'un tuyau de sortie ou d'un cône de sortie à un sac contenant un liquide

(30) Priority: 18.06.1987 GB 8714303
(43) Date of publication of application: 21.12.1988
(73) Proprietor: E.R. Squibb & Sons, Inc., Princeton, N.J. 08540-4000 (US)
(72) Inventor: Steer, Peter Leslie, East Grinstead Sussex (GB)
(74) Representative: Cook, Anthony John

(56) References cited:
- DE-A- 2 249 406
- DE-A- 3 401 262
- FR-A- 2 076 932
- GB-A- 871 820
- GB-A- 1 332 447
- GB-A- 2 126 483

## Description

Fluid containers or bags designed for medical applications conventionally include an outlet pipe or tap to drain the container. One such type of bag is a urostomy pouch such as shown by Steer et al. in US-A-4,300,560 having a valve assembly welded in place. Another type of valve assembly including an O-ring is shown by Steer in GB-A-2,191,757.

Other types of urine collecting bags including an outlet pipe or tap are collection receptacles of a urinary incontinence system such as the leg bag shown by Steer et al. in US-A-4,462,510 and the hanging bag shown by Steer et al. in US-A-4,534,766.

This invention relates to a method of assembly in the manufacture of a bag for containing liquids and to securing an outlet pipe or tap to a bag for containing liquids.

German Published Application No (DE-A) 3401 262 and its U.K. counterpart GB-B-2 135 652 show an access port forming device and a method of forming an access port. This is an access port into the interior of an already manufactured bag. During use of this method, nested generally conical members (13" and 15" in Fig 8 of GB-B-213S652) are employed. These documents teach the use of piercing element 14 to make the aperture which becomes the access port. In contrast, the present invention relates to the manufacture of a bag.

French Patent No. (FR-A) 2076932 shows a urine collecting device wherein a tapering member 64 (fig 5 of '932) surrounds the penis 60 of the wearer and the upper (open) end of a condom 62 is trapped by the lower end 66 of the tapering member. In this way, the condom is securely attached to the wearer and twisting of it is avoided. This patent also shows in Figs 1 and 2 a condom trapped between a member 2 and a tapering member 12. The present invention is not concerned with devices to be worn on the penis.

According to the invention, there is provided a method of assembly in the manufacture of a bag for containing liquid, the bag including an outlet region trapped in a leakproof manner between a nested pair of complementarily tapered members, the method being characterized by (a) employing a nested pair of members comprising inner and outer funnel members having, over their nested portions, outwardly tapered portions and parallel portions; (b) trapping said bag outlet region between said inner and outer funnel members in the area of said outward tapers with said parallel portion of said outer funnel member extending below the parallel portion of said inner funnel member by pushing the inner funnel member axially relative to the outer funnel member until it is fully nested and an O-ring sealing means becomes lodged substantially within a recess in the inner surface of the outer member or the outer surface of the inner member as the case may be; and (c) securing, by heat welding, RF or ultrasonic welding, the outlet region of the bag to the inner tunnel.

The outer funnel may have an annular recess, of a cross-sectional shape complementary to the O-ring sealing means, in its inner surface. This recess locates the O-ring sealing means definitely when one funnel is moved relative to the other.

The outlet region of the bag is permanently secured by heat welding, RF welding or ultrasonic welding, to the inner funnel.

The O-ring sealing means may be located on a tapering portion or on a parallel-sided portion of the funnel, the latter being preferred.

The invention will be better understood from the following non-limiting description of two examples thereof given with reference to the accompanying drawings, in which: -
Figure 1 is a diagrammatic sketch cross sectional view of a bag for containing liquid;
Figures 2 - 4 are cross sections illustrating the preferred embodiment of the invention; and
Figure 5 is a scrap section showing an alternative in which an O-ring sealing means is molded in position in an annular channel in the external surface of the inner funnel.

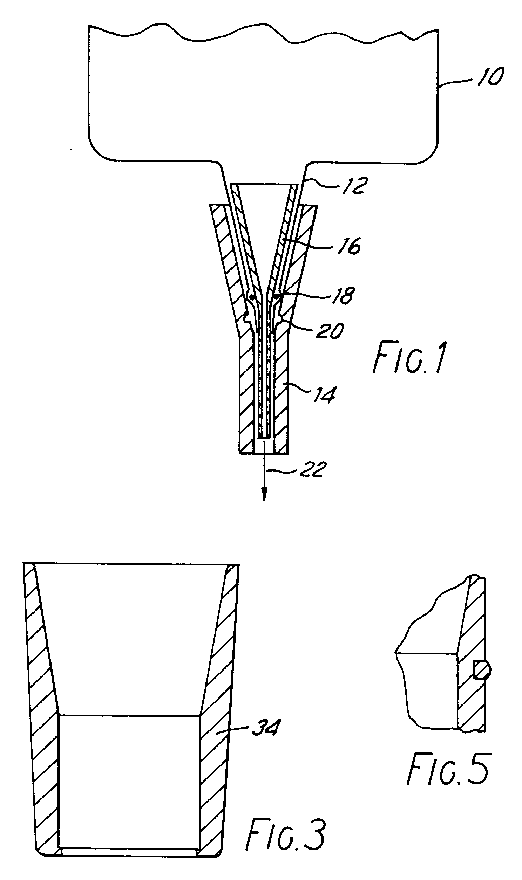

As shown in Figure 1, the bag 10 for containing liquids includes an outlet region 12 whose walls are trapped between outer funnel 14 and inner funnel 16. An O-ring 18 is provided to be received in annular recess 20 in the inner wall of outer funnel 14.

The parts are shown in their relative positions just prior to final assembly, which is effected by pushing the funnel 16 axially relative to the funnel 14 so that it is fully nested and the O-ring 18 is lodged substantially within the recess 20. The liquid exit path is shown by the arrow 22. As will be seen, the only theoretically available leakage path is down the outer surface of the inner funnel, past the compressed O-ring, and up the inner surface of the outer funnel. Leakage is hence virtually impossible.

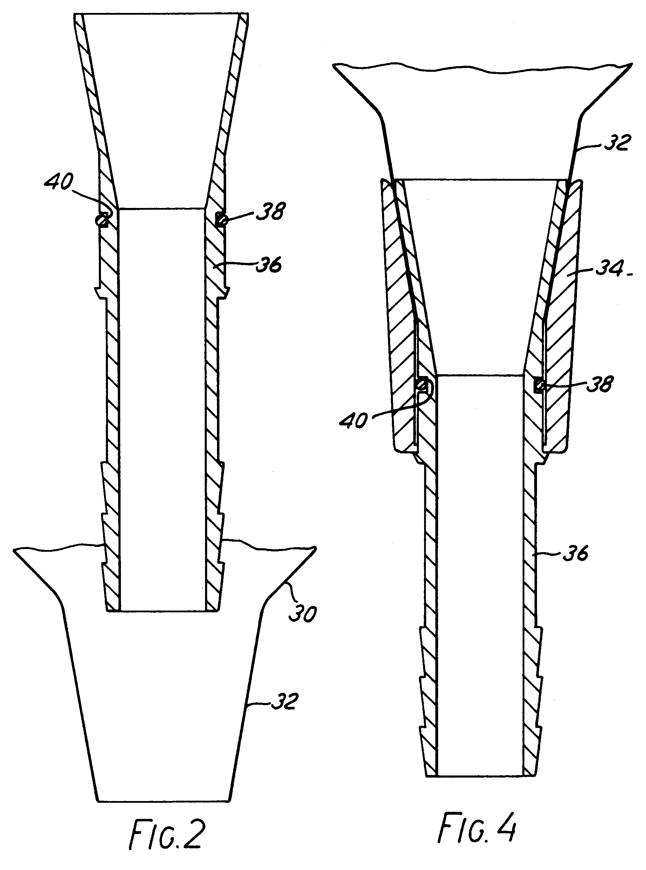

The parts of the embodiment of the invention shown in Figures 2 - 4 are as follows. This embodiment employs the same principle as the Figure 1 embodiment. The bag 30 for containing liquid has an outlet region 32. An outer funnel 34 and an inner funnel 36 are provided between which the bag walls of outlet region 32 are trapped. An O-ring sealing means 38 is located on the parallel-sided portion of inner funnel 36 beneath the outward tapered region. The O-ring 38 is accomodated within an annular recess 40 in the outer wall of inner funnel 36. The portion of the inner funnel 36, as shown in Figure 4, which extends beyond the outer funnel 34 when the parts are assembled includes a series of saw-teeth on its outer wall to permit tubing to be slid over this portion of the inner funnel.

As seen in Figure 5, instead of a separate O-ring, one can be initially molded in position in the annular channel or recess.

The funnels may be made of any suitable material, e.g., a synthetic plastics material such as polyester. This may be molded. In some circumstances a pair of metal funnels may be employed.

As will be seen from the foregoing description, on the inner funnel molding there is fitted a parallel exit tube below the tapering portion. An O-ring is disposed around the parallel portion or there may be an injection molded soft ring. The bag film finishes on the taper, and the two components squash the O-ring in between them so that there cannot be a leak path at that point. The purpose and advantage of the taper is that the very action of pushing on the outer funnel to deform the sealing means on the inner funnel tends to force the bag material up the taper thus tightening its grip around the molding.

The upper portions of bags 10 and 30 are not shown. These upper portions could, for example, be of a urostomy configuration as shown in US-A-4,300,560 or of a leg bag configuration as shown in US-A-4,462,510.

While the term funnel has been employed in this description, it will be appreciated that co-operating parts of non-circular cross section might be employed as an alternative.

## Claims

1. A method of assembly in the manufacture of a bag for containing liquid, the bag including an outlet region (12) trapped in a leakproof manner between a nested pair of complementarily tapered members, the method being characterized by (a) employing a nested pair of members (14, 16) comprising inner (16) and outer (14) funnel members having, over their nested portions, outwardly tapered portions and parallel portions; (b) trapping said bag outlet region (12) between said inner (16) and outer (14) funnel members in the area of said outward tapers with said parallel portion of said outer funnel member (14) being shorter than the parallel portion of said inner funnel member (16) by pushing the inner funnel member (16) axially relative to the outer funnel member (14) until it is fully nested and an O-ring sealing means (18 or 38) becomes lodged substantially within a recess (20 or 40) in the inner surface of the outer member or the outer surface of the inner member as the case may be; and (c) securing, by heat welding, RF or ultrasonic welding, the outlet region (12) of the bag to the inner funnel (16).

## Patentansprüche

1. Zusammenbauverfahren bei der Herstellung eines Beutels für die Aufnahme von Flüssigkeit, wobei der Beutel einen zwischen einem ineinandergreifenden Paar komplementär schräg verlaufender Elemente auf dichte Weise eingeschlossenen Auslaßbereich (12) aufweist, wobei das Verfahren gekennzeichnet ist durch (a) Verwenden eines ineinandergreifenden Paars von Elementen (14, 16) mit inneren (16) und äußeren Trichterelementen (14) mit, über den ineinandergreifenden Abschnitten, nach außen schräg ver laufenden Abschnitten und parallelen Abschnitten; (b) Einschließen des Auslaßbereichs (12) des Beutels zwischen das innere (16) und das äußere Trichterelement (14) im Bereich der Außenschrägen wobei der parallele Abschnitt des äußeren Trichterelements (14) kürzer ist als der parallele Abschnitt des inneren Trichterelements (16), durch Drücken des inneren Trichterelements (16) radial relativ zum äußeren Trichterelement (14), bis es völlig umschlossen wird, wobei eine O-Ring-Dichtung (18 oder 38) im wesentlichen in einer Vertiefung (20 oder 40) in der Innenfläche des äußeren Elements bzw. in der Außenfläche des inneren Elements aufgenommen wird; und (c) Befestigen des Auslaßbereichs (12) des Beutels durch Heißverschweißen, HF-oder Ultraschall-Schweißen am inneren Trichter (16).

## Revendications

1. Procédé d'assemblage dans la fabrication d'un sac destiné à contenir un liquide, le sac comportant une région de sortie (12) emprisonnée de façon étanche entre deux éléments tronconiques complémentaires emboîtés l'un dans l'autre, ce procédé étant caractérisé par (a) l'utilisation de deux éléments emboîtés (14,16) consistant en des entonnoirs intérieur (16) et extérieur (14) ayant chacun, sur leur partie emboîtée, une partie conique vers l'extérieur et une partie parallèle; (b) l'emprisonnement de ladite région de sortie (12) du sac entre lesdits entonnoirs intérieur (16) et extérieur (14) dans la zone desdites parties coniques vers l'extérieur, ladite partie parallèle dudit entonnoir extérieur (14) étant plus courte que la partie parallèle dudit entonnoir intérieur (16), en poussant l'entonnoir intérieur (16) axialement par rapport à l'entonnoir extérieur (14) jusqu'à ce qu'il soit complètement emboîté et qu'un joint torique d'étanchéité (18 ou 38) vienne se loger sensiblement à l'intérieur d'un évidement (20 ou 40) dans la surface intérieure de l'entonnoir extérieur ou dans la surface extérieure de l'entonnoir intérieur, suivant le cas; et (c) la fixation, par thermosoudage, soudage RF ou soudage par ultrasons, de la région de sortie (12) du sac à l'entonnoir intérieur (16).
